# EUROPEAN PATENT SPECIFICATION

(11) **EP 3 099 190 B1**
(45) Date of publication and mention of the grant of the patent: **14.11.2018**
(21) Application number: 15702557.8
(22) Date of filing: 28.01.2015
(51) Int. Cl.: A24F 47/00, A61M 11/04, A61M 15/06

(54) **AEROSOL-FORMING MEMBER**
AEROSOLBILDENDES ELEMENT
ÉLÉMENT DE FORMATION D'AÉROSOL

(30) Priority: 29.01.2014 GB 201401520
(43) Date of publication of application: 07.12.2016
(73) Proprietor: Batmark Limited, London WC2R 2PG (GB)
(72) Inventor: BUCHBERGER, Helmut, 4490 St. Florian (AT); DICKENS, Colin John, London WC2R 3LA (GB); FRASER, Rory, London WC2R 3LA (GB)
(74) Representative: D Young & Co LLP
(86) International application number: PCT/GB2015/050195
(87) International publication number: WO 2015/114328

(56) References cited:
- EP-A1- 2 340 729
- WO-A1-2013/057185
- US-A1- 2011 226 236
- US-A1- 2013 081 623

## Description

### Field of the Invention

The invention relates to an aerosol-forming member for an aerosol delivery device. The invention also relates to an aerosol delivery device component comprising the aerosol-forming member according to the invention, and an aerosol delivery device comprising said aerosol delivery device component.

### Background

An aerosol delivery device is a device used for delivering substances into the body via the lungs. One type of aerosol delivery device forms a vapour of a solution in which the substances are dissolved. This vapour condenses within the aerosol delivery device as it mixes with air so as to form droplets or aerosol which is suitable for inhalation. These aerosol delivery devices may comprise a heating element that is configured to evaporate the solution held within the aerosol delivery device so as to form said aerosol. Alternatively, some aerosol delivery devices may utilise piezo atomizers to generate the aerosol.

Document WO 2013/057185 A1 discloses an aerosol-forming member for an aerosol delivery device.

### Summary

According to the invention, and as defined in claim 1, there is provided an aerosol-forming member comprising a sheet of material configured to heat and wick a solution, wherein the sheet of material comprises at least one corrugation.

According to the invention the sheet of material comprises a capillary structure configured to wick a solution.

The capillary structure may be exposed on both sides of the sheet of material. The capillary structure may extend throughout the whole sheet of material. The sheet of material may be heatable. The capillary structure may be made of an (electrically) heatable material.

In an alternative embodiment, the sheet of material may comprise a first layer that is heatable and a second layer comprising a capillary structure.

The sheet of material may comprise a peak and a trough, with said peaks/troughs being either rounded or pointed, i.e. where the sheet of material includes corrugations forming vertices.

Alternatively, the sheet of material may have a wave form.

According to another aspect of the invention, there is provided an aerosol delivery device component comprising an air inlet and an air outlet fluidly communicating via an aerosol chamber defined by chamber walls, and an aerosol-forming member as described above, the aerosol forming member being located at least partially within the aerosol chamber.

In one embodiment, the aerosol delivery device component may further comprise an aerosol-forming member located completely in the aerosol chamber.

In one embodiment, the sheet of material may comprise two opposing major surfaces that are aligned with a direction of flow of air through the aerosol chamber.

In another embodiment, the sheet of material may comprise two opposing ends that are attached to the aerosol delivery device component such that the sheet of material is suspended across the aerosol chamber.

The aerosol forming member may be attached to at least one of the chamber walls.

In one embodiment, one of the chamber walls is a printed circuit board and the aerosol-forming member is attached to the printed circuit board.

In one embodiment, the at least one corrugation of the sheet of material may be in close proximity to at least one of the chamber walls.

In one embodiment, the at least one corrugation of the sheet of material may be in contact with at least one of the chamber walls.

At least one of the chamber walls may comprise a heat shield.

According to yet another aspect of the invention, there is provided an aerosol delivery device component comprising an air inlet and an air outlet fluidly communicating via an aerosol chamber defined by chamber walls, wherein at least one of the chamber walls comprises a liquid reservoir matrix.

The liquid reservoir matrix may comprise a capillary structure.

In one embodiment, the liquid reservoir matrix may comprise a heat resistant layer and a resilient layer.

In another embodiment, two opposing chamber walls may each comprise a liquid reservoir matrix, and the heat resistant layer of each liquid reservoir matrix is innermost relative to the aerosol chamber such that the resilient layer urges the heat resistant layers towards one another.

The aerosol delivery device component may further comprise an aerosol-forming member located at least partially within the aerosol chamber. In one embodiment, the aerosol delivery device component may further comprise an aerosol-forming member located completely within the aerosol chamber.

In one embodiment, the aerosol-forming member may comprise any of the features described above.

The at least one corrugation of the sheet of material may be in contact with the liquid reservoir matrix.

According to a further aspect of the invention, there is provided an aerosol delivery device comprising an aerosol delivery device component and/or aerosol forming member as described above.

According to the invention, there is provided an aerosol-forming member comprising a sheet of material configured to heat and wick a solution, wherein the sheet of material has a cross-sectional profile having at least one point of inflection.

### Brief Description of the Drawings

Embodiments of the invention will now be described, by way of example only, with reference to the accompanying drawings in which:
Figure 1a shows a cross-sectional side view of an aerosol delivery device comprising an aerosol-forming member according to an embodiment of the invention;
Figure 1b shows a cross-sectional side view of an alternative aerosol delivery device comprising an aerosol-forming member according to an embodiment of the invention;
Figure 2a shows a cross-sectional side view of a detachable component, which may be used in the device of Figure 1a, comprising an aerosol-forming member according to an embodiment of the invention;
Figure 2b shows a cross-sectional view of the aerosol delivery device along the line X-X of Figure 2a;
Figure 2c shows a cross-sectional view of the aerosol delivery device along the line X-X of Figure 1b;
Figure 3a shows a cross-sectional view of another embodiment of the aerosol delivery device; and
Figure 3b shows a cross-sectional view of another embodiment of the aerosol delivery device.

### Detailed Description

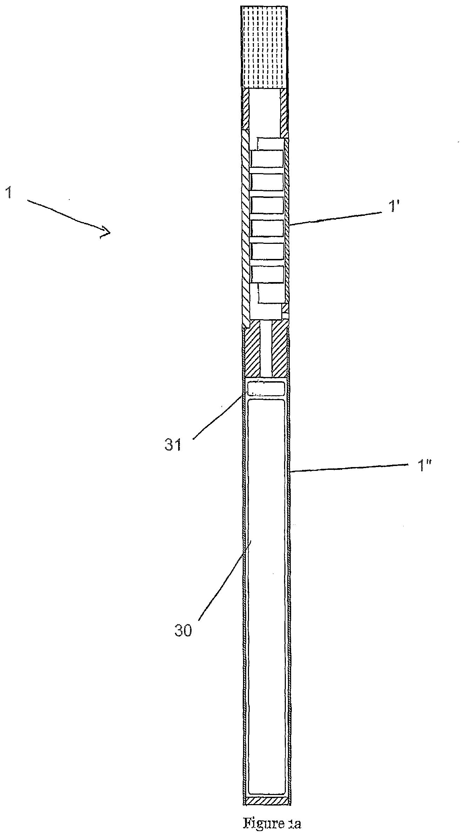

Referring now to figure 1a, an aerosol delivery device 1 according to the present invention is disclosed. The aerosol delivery device comprises an aerosol delivery device component 1', and an energy store component 1". The aerosol delivery device component 1' is removably attachable to the energy store component 1", however it is envisaged that in an alternative embodiment, the aerosol delivery device component 1' and the energy store component 1" are inseparable such that they form a single component.

The aerosol delivery device component 1' may be disposable and the energy store component 1" may be reusable. However, it is envisaged that when the two components are formed as a single component then the aerosol delivery device may be disposable or reusable.

The energy source component 1" comprises a housing holding a battery 30 and an electric circuitry 31 as shown in figure 1a. It should be appreciated that an alternative power source to a battery may be used.

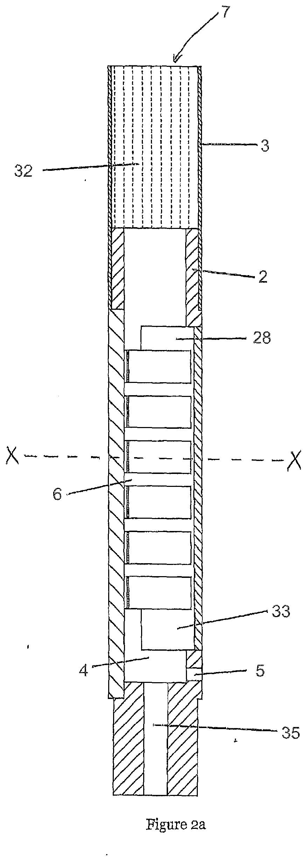

The aerosol delivery device component 1' is shown in greater detail in Figure 2a and it comprises a housing 2 formed with a mouthpiece 3 at one end and an attachment end formed with a connecting passage 35 at the opposite end. The connecting passage 35 electrically connects components held in the aerosol delivery device component 1' with the battery 15 disposed in the energy store component 1" via the electric circuitry 31 (not shown).

The housing 2 is further formed with an air passage extending through the aerosol delivery device component 1'. The air passage comprises an air inlet 5, plenum chamber 4, chamber inlet 33, aerosol chamber 6, chamber outlet 28 and outlet aperture 7. In use air is drawn in through the air inlet 5, into the plenum chamber 4, then to the chamber inlet 33 which supplies the air into the aerosol chamber 6, the air then exits the aerosol chamber 6 via chamber outlet 28 and leaves the aerosol delivery device component 1' via the outlet aperture 7 formed in the mouthpiece 3.

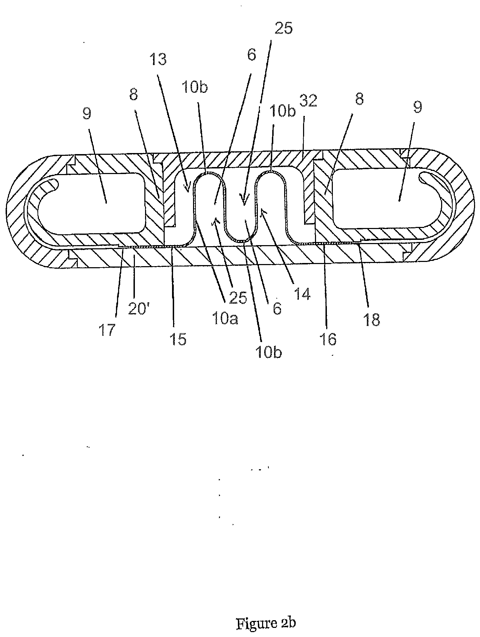

Figure 2b illustrates a cross-sectional view of the aerosol delivery device component 1' along line X-X shown in Figure 2a. As can be seen in Figure 2b, the aerosol chamber 6 is located in the centre of the housing and is defined by chamber walls. The chamber walls comprise two partitioning walls 8, a chamber side wall 32 and a support plate 20' as explained in more detail below. An aerosol-forming member 10A according to an embodiment of the invention is located in the aerosol chamber 6. On opposite sides of each partitioning walls 8 relative to the aerosol chamber 6, are two solution reservoirs 9 configured to contain a solution. In one embodiment the support plate 20' is the PCB. In an alternative embodiment, the support plate 20' is integrated with the housing 2 such that a capillary gap is formed between the housing and the partitioning wall 8.

According to one embodiment of the invention, the aerosol-forming member 10A may comprise a sheet of material having a single layer that is configured to wick and heat a solution. Thus, the sheet of material can absorb solution from the solution reservoirs 9 and thereafter heat it up so that it evaporates and forms a vapour. The sheet of material is sheet-like in nature. The sheet of material may comprise an open-pored structure, foam structure, mesh structure or interconnecting network of pores, all of which form a capillary structure. The capillary structure enables the aerosol-forming member 10A to wick or absorb a solution. The *term "capillary structure"* used herein is to be understood as a structure through which liquid or a solution can travel as a result of capillary action.

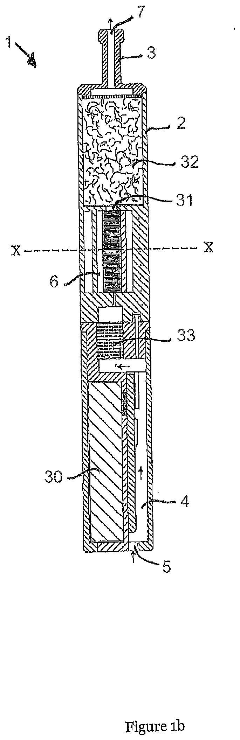

An embodiment of an alternative aerosol delivery device 1 is shown in figure 1b and it comprises a housing 2 with a mouthpiece 3. A passage 4 is provided in the housing 2 and is open to atmosphere via an air inlet 5. The passage 4 is in fluid communication with an aerosol chamber 6 which in turn is in fluid communication with an outlet aperture 7 formed in the mouthpiece 3. Therefore, in use, air may be drawn through the air inlet 5 and into the aerosol chamber 6, via the passage 4, and then through the outlet aperture 7 as is indicated by the arrows in figure 1.

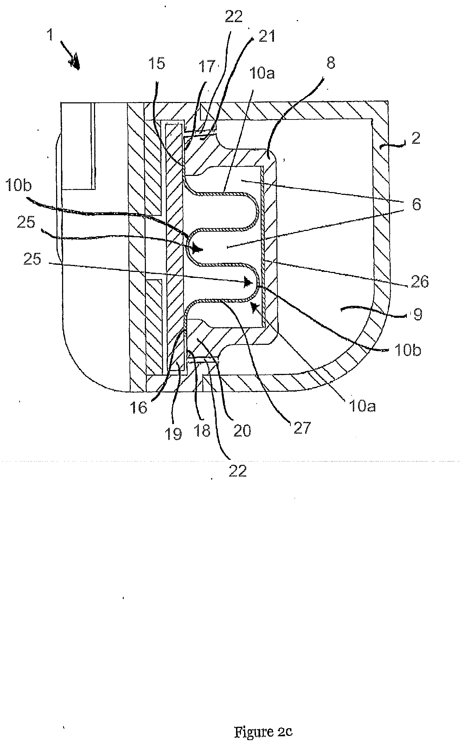

Referring now to figure 2c which shows a cross-sectional view of the aerosol delivery device 1 of figure 1b, a space is provided inside the housing 2 which is divided by a partitioning wall 8 into the aerosol chamber 6 and a liquid reservoir 9 that contains a solution. The aerosol chamber 6 is defined by the partitioning wall 8, a support plate 19 and a heat shield 26. The partitioning wall 8, support plate 19 and heat shield 26 act as chamber walls. Two capillary gaps 17,18 are formed between the ends 20, 21 of the partitioning wall 8 and the support plate 19. For the avoidance of doubt, the device shown in figure 1 may also include a similar heat shield as necessary. In one embodiment the support plate 19 is the PCB. In an alternative embodiment, the support plate 19 is integrated with the housing 2 such that a capillary gap is formed between the housing and the partitioning wall 8.

An aerosol-forming member 10a according to an embodiment of the invention is located in the aerosol chamber 6 as seen in figure 2b or 2c. The aerosol-forming member 10a comprises a sheet of configured to have corrugations 10b such that it comprises peaks and troughs. In the present application, a corrugation is to be understood as two bends, or a peak and a trough, or a ridge and a groove. The sheet of material may also be described as having a cross-sectional profile comprising at least one point of inflection.

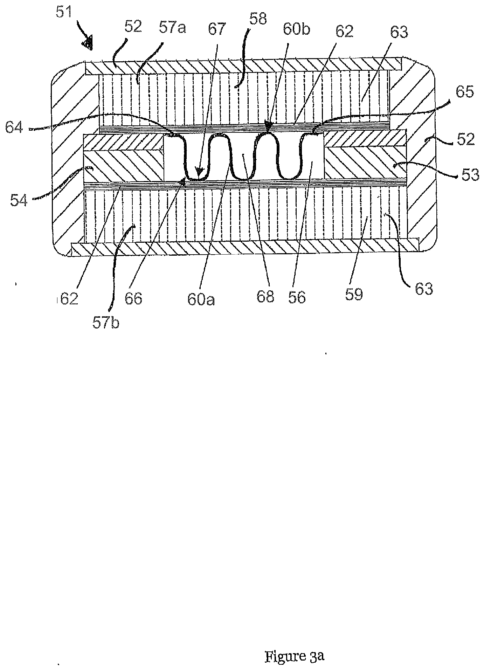

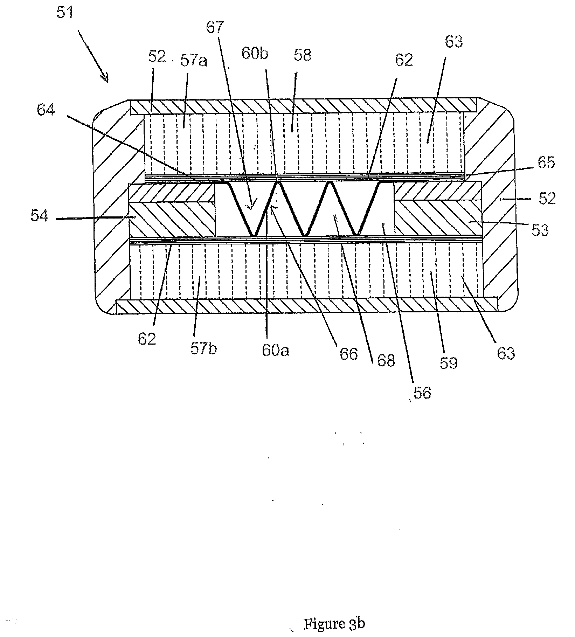

The corrugations of the aerosol-forming members 10a may follow a meandering or oscillating path, or a sine curve or any other similar pattern. The aerosol-forming member 10a may comprise regular corrugations such that it comprises a repetition of identical corrugations as seen in figure 2. However, in an alternative un-illustrated embodiment the aerosol-forming member may comprise irregular corrugations wherein the shape of the peaks and troughs differ from one another. Although figures 2a and 2b show an aerosol-forming member 10a comprising rounded corrugations 10b, i.e. rounded peaks and troughs, it should be understood that the invention is not limited thereto, but also includes corrugations forming vertices (forming zig-zag like corrugations). These vertices may have obtuse, acute and/or right angles. Such an arrangement is shown in the embodiment of figure 3b (the description of the corresponding reference numerals for the embodiment of figure 3a applying also to the embodiment of figure 3b).

The sheet of material may comprise a single layer that is sheet-like in nature and comprises two major opposing surfaces 13, 14 and two opposing ends 15, 16. The two opposing ends 15, 16 are attached to a support plate 19 (according to the device of figure 1b) or support plate 20' (according to the device of figure 1a) such that they locate in corresponding capillary gaps 17, 18 formed between the support plate 19, 20' and the ends 21, 20 (according to the device of figure 1b) of the partitioning wall 8. Preferably the two opposing ends 15, 16 are also electrically connected to the support plate 19. The support plate 19 may be a printed circuit board (PCB). The sheet of material is configured to extend in a contact-free manner across the aerosol chamber 6 such that the aerosol-forming member 10a is suspended across the aerosol chamber 6 and only the ends 15, 16 of the aerosol-forming member 10a are in contact with the chamber walls of the aerosol chamber 6. This configuration reduces undesired loss in heat conduction of the aerosol-forming member 10a. As a result, the aerosol-forming member 10a can heat up faster to a sufficient temperature wherein a solution held in the aerosol-forming member evaporates than if the aerosol-forming member was in continuous contact with the chamber walls. It should be understood that the sheet of material does not have to be a single layer, but can be a sheet of multiple layers of the same or different material that are layered/laminated/attached so as to form the final sheet-like material.

In one embodiment, the aerosol-forming member 10a has corrugations such that it extends substantially across the entire cross-section of the aerosol chamber 6 as seen in figures 2b,c and 3a,b. More specifically, the aerosol-forming member 10a does not only extend in a direction between the capillary gaps 17, 18, but also extends across substantially the whole distance between the support plate 19 and the partitioning wall 8/heat shield 26 (according to the embodiment of figure 2c), and across substantially the whole distance between the support plate 20' and the chamber wall 32/heat shield 26 (according to the embodiment of figure 2b). Thus, in these embodiments, the corrugations 10b are adjacent or in close proximity to the chamber walls, or more specifically the wall 8, 32, the heat shield 26 and the support plate 19,20'. In another embodiment, the aerosol-forming member 10a extends across the aerosol chamber 6 to such an extent that the corrugations 10b contact or engage with the chamber walls (as shown in figures 3a and 3b). In yet another embodiment, the depth of the corrugations 10b is shorter such that the aerosol-forming member 10a does not extend across the whole distance between the support plate 19 and the partitioning wall 8/heat shield 26 (according to the embodiment of figure 2c), or the aerosol-forming member 10a does not extend across the whole distance between the support plate 20' and the wall 32/heat shield 26 (according to the embodiment of figure 2b). It is not necessary that each of the corrugations 10b contact or be in close proximity with the chamber walls. Where there are multiple corrugations, each corrugation may be independently configured to either be in contact with the chamber walls, be in close proximity with the chamber walls, or to be distanced from the chamber walls.

The sheet of material, which may comprise a single layer, is configured to wick and heat a solution such that the sheet of material can absorb solution and thereafter heat it up such that it evaporates and forms a vapour. The sheet of material may comprise an open-pored structure, foam structure or interconnecting network of pores, all of which form a capillary structure. The capillary structure enables the aerosol-forming member 10a to wick or absorb a solution. The term *"capillary structure"* used herein is to be understood as a structure through which liquid or a solution can travel as a result of capillary action.

The aerosol-forming member 10a may be made of a porous granular, fibrous or flocculent sintered metal(s) so as to form said capillary structure. In another embodiment, the aerosol-forming member 10a comprises an open-pored metallic foam or a group of layers of wire mesh or calendered wire mesh or wire fabric which also forms a capillary structure. The aerosol-forming member 10a may be formed from stainless steel, e.g. AISI 304 or 316, or from a heating conductor alloy, like nickel chromium alloys.

Furthermore, the aerosol forming member 10a may be formed with a capillary structure that extends throughout the whole aerosol-forming member 10a such that it is exposed on the two major surfaces 13, 14 of the sheet of material. In this embodiment each of the major surfaces would be exposed to the chamber 6. Alternatively, one of the major surfaces 13, 14 may optionally be sealed with a metallic foil or cover that is sintered or attached to said major surface such that said surface is vapour impermeable. Alternatively, a region of one or both of the major surfaces 13, 14 may be sealed so as to be vapour impermeable. In another embodiment, the aerosol-forming member 10a is configured such that the capillary structure does not extend throughout the whole aerosol-forming member. For example, in one embodiment the capillary structure does not extend to one of the major surfaces 13, 14 such that there is no exposed capillary structure in this area. Alternatively the capillary structure is exposed on a region of one or both major surfaces 13, 14. In the context of the present disclosure, reference to "exposure" of the capillary surface does so with reference to the chamber 6 and it is possible that there are areas of the capillary surface that extend to the major surfaces 13, 14 but that are covered by other components of the device.

In yet another un-illustrated embodiment, a thin support layer may be attached to one or both of the major surfaces 13, 14. Such a support layer provides stability to the sheet of material. The support layer may be formed from a wire mesh or from individual wires and may be made of stainless steel.

The material from which the aerosol-forming member 10a is formed is heatable in that it comprises sufficient electrical resistivity so that when current is passed through, the aerosol-forming member 10a heats up to a temperature sufficient to cause the solution held in the capillary structure to evaporate or vaporise. In these embodiments, the aerosol-forming member 10a can be considered to comprise a heating element formed with a capillary structure such that the heating element and the capillary structure are integrated and form a single entity or unit.

In the above described embodiments wherein the sheet of material comprises a single layer configured to wick and heat a solution, the sheet of material can be described as comprising a heating element and a wick that are arranged in the same surface.

In an alternative un-illustrated embodiment, the sheet of material may comprise a plurality of layers, for example it may comprise any combination of the aforementioned structures and materials, e.g. by providing multiple layers of different structures/ materials, the layers being joined together, e.g. by sintering. One such alternative un-illustrated embodiment will now be described in more detail.

This un-illustrated alternative aerosol-forming member comprises a sheet of material that is sheet-like in nature and formed from a plurality of layers. For example, the aerosol-forming member 10a may comprise a first heatable layer acting as a heating element. The first layer is formed from a material that is configured to be heated up and may comprise a metal wire mesh, it may be made of stainless steel or nickel chromium alloys. The aerosol-forming member 10a may further comprise a second layer formed with an open-pored structure, foam structure or interconnecting network of pores, all of which form a capillary structure. The capillary structure enables the aerosol-forming member 10a to wick or absorb a solution. This second layer may comprise a fibre web or fabric made of glass fibres, glass fibre yarns or any other non-conductive and inert, thus relatively non-heatable (electrically non-heatable) fibre materials. In this embodiment the sheet of material can be described as comprising a heating element and a wick that are arranged in parallel surfaces and are connected to each other. The second layer acts as a wick.

The first layer (heating element) and the second layer (wick formed with a capillary structure) are laid on top of each other so as to form a sheet of material having two opposing major surfaces, wherein the capillary structure is exposed on one or both major surfaces (depending on the configuration of the heating element).

In an alternative un-illustrated embodiment, the sheet of material comprises a third layer that is similar to the second layer in that it comprises a capillary structure. The second and the third layer sandwich the first layer such that the capillary structure is exposed on both major surfaces of the sheet of material.

In the embodiments wherein the sheet of material is formed from a plurality of layers as described above, the first layer forming the heating element and the second and/or third layer(s) forming the wick are parallel and connected to each other. The layers may be connected to each other by mechanical, chemical or thermal means. In one embodiment, the layers are sintered or fritted to one another.

It should be understood that the present invention wherein the sheet of material comprises a plurality of layers is not limited to the examples described above. For example, in an alternative embodiment both the first and the second layers may be made of a heatable material. For instance, the first layer may comprise a metal foil, and the second layer may be made of a porous granular, fibrous or flocculent sintered metal(s) or comprise an open-pored metallic foam or a wire mesh structure all of which form said capillary structure. The first and second layers may be formed from stainless steel and may be sintered together. In this embodiment the sheet of material can be described as comprising a heating element and a wick that are arranged in the same surface and in parallel surfaces, the capillary structure being only exposed on one of the major surfaces of the sheet of material.

In another embodiment, the first and second layers may be made of porous heatable material(s), such that both layers are configured to heat and wick a solution. In this embodiment the sheet of material can be described as comprising a heating element and a wick that are arranged in the same surface and in parallel surfaces.

In a further alternative un-illustrated embodiment, the sheet of material comprises a porous first layer having small sized pores and a second porous layer having larger sized pores than the first layer, thus both layers are formed with a capillary structure however the second layer forming the inner major surface can emit more vapour than the first layer forming the outer major surface. At least one of the two layers is formed from a heatable material as described above. Both layers may be formed with a structure and material as discussed above in relation to the capillary structure.

The sheet of material according to any of the above described embodiments has a thickness or depth that falls within the range of 20-500µm. Alternatively, the thickness falls within the range of 50 to 200µm. The thickness or depth should be understood as meaning the distance between the two major surfaces 13, 14 of the sheet of material.

The ends 21, 20 of the partitioning wall 8 (according to the embodiment of figure 2c) are formed with two supply passages 22 such that the reservoir 9 and capillary gaps 17, 18 are in fluid communication. The supply passages are of a width sufficient to achieve a capillary effect. Therefore, in use, a solution held in the reservoir 9 moves by capillary action from the fluid reservoir 9 into the supply passages 22 towards the capillary gaps 17, 18, and then from the capillary gaps 17, 18 to the capillary structure at the ends 15, 16 of the aerosol-forming member 10a. The capillary structure of the aerosol-forming member 10a provides a capillary effect similar to a wick, thus the capillary structure enables the aerosol-forming member 10a to absorb the solution provided to the capillary gaps 17, 18 such that the solution is distributed throughout the whole capillary structure of the sheet of material.

The embodiment of figure 2b operates in a similar manner, except the aerosol-forming member 10a is fed from reservoir 9 via capillary gaps 17, 18.

It should be understood that the present invention is not limited to two capillary gaps 17, 18, but may comprise only a single capillary gap feeding only one of the ends 15,16 of the aerosol-forming member 10a.

A battery 30 controlled by a controller forming part of a printed circuit board (PCB) is disposed in the housing 2 as seen in figure 1, and the ends 15, 16 of the aerosol-forming member 10a are electrically connected, e.g. via circuitry, to the positive and negative terminals of the battery 30 respectively. When current is drawn from the battery 30 and through the sheet of material, the resistance of the sheet of material causes it to increase in temperature. In the embodiment wherein the sheet of material comprises a non-porous heatable first layer, for instance a metal foil, and where the outer major surface is formed from said first layer, the resistance of said first layer causes the first layer acting as a heating element to increase in temperature, the first layer in turn heating up adjacent second and/or third layers including the solution contained/stored in the pores/void of the capillary structure of said second and/or third layers. The current drawn by the battery 30, and thus the temperature of the sheet of material may be controlled by a switching circuit, e.g. a Power-MOSFET switching circuit, within the housing 2. The switching circuit may provide automatic control of the temperature, for example, by using temperature sensors (not shown), or may be controlled by a button or dial (not shown) provided on the housing 2 that may be manipulated by the user.

In one embodiment the partitioning wall 8 may be provided with a heat shield 26 on a surface facing the aerosol-forming member 10a. The heat shield 26 is shown in figure 2b and it protects the partitioning wall 8 from overheating as the temperature of the aerosol-forming member 10a is increased. The heat shield 26 may be formed from a thin non-conductive material like oxidised stainless steel wire mesh or inert fabrics like glass or carbon fabrics. It should be understood that the heat shield 26 is optional. Operation of the aerosol delivery device will now be described with reference to figures 1 and 2. In use, the user may manually activates the aerosol delivery device 1 or the aerosol delivery device 1 may be activated automatically as the user starts puffing on the aerosol delivery device 1. This may be achieved by a pressure sensor (not shown) included in the electric circuitry 31 and communicating with the inlet passage/plenum chamber 4 via a connecting passage. In either embodiment, the battery 30 provides a potential difference between the opposing ends 15, 16 of the aerosol-forming member 10a as the aerosol delivery device is activated, causing current to flow though the sheet of material such that the member 10a increases in temperature. This increase in temperature causes the solution held in the capillary structure of the sheet of material to evaporate so as to form a vapour. The evaporated solution mixes with the air drawn into the aerosol delivery device via passage 4 by the user. The evaporated solution mixes with air in the aerosol chamber 6, and as this occurs the vapour condenses and forms droplets such that an inhalable aerosol is produced.

The aerosol-forming member according to any of the above described embodiments is located in the housing 2 such that the planes of the major surfaces 13, 14 are parallel to or substantially aligned with the direction of the airflow through the aerosol chamber 6. Thus, when a solution is held in the aerosol-forming member 10a and it is heated up such that the solution evaporates, the solution evaporates in a direction transverse to the direction of the airflow. In the embodiments wherein the capillary structure is exposed on both sides of the sheet of material, the solution is evaporated from both sides in opposite directions. The corrugations 10b of the sheet of material form channels 25 through which air flows and also in which a solution is evaporated such that when it mixes with airflow aerosol is formed. Thus, the corrugations 10b or channels 25 direct the flow of aerosol through the aerosol delivery device towards the user. Furthermore, as a result of the corrugations 10b, solution is evaporated from the major surfaces 13, 14 in a direction towards another region of the major surfaces 13, 14 which results in reduced levels of aerosol condensing on the chamber walls and other internal components. Furthermore, as vapour is emitted from the major surfaces 13, 14 towards another region of the same major surface, vapour density is increased. Additionally, as the aerosol-forming member cools down aerosol and vapour remaining in the aerosol chamber 6 that condenses onto one of the major surfaces 13, 14 will be reabsorbed into the capillary structure of the aerosol-forming member and re-evaporated as the aerosol-forming member heats up again. Condensate formed on the chamber walls may at least partially be reabsorbed into the capillary structure via the corrugations 10b.

As previously described, the corrugated configuration reduces condensation from forming and accumulating on the chamber walls, internal components and/or inner walls of the housing 2. Thus, sponges or other means for absorbing condensation not inhaled by the user that are used in some conventional aerosol delivery devices may be omitted. This results in a more compact aerosol delivery device 1, as well as a simplified manufacturing process and reduced costs. Furthermore, by reducing the amount of aerosol and vapour from condensing onto inner walls of the housing 2, the transfer of condensation heat to the housing 2 may be reduced, making the aerosol delivery device 1 more comfortable for the user to hold.

After the aerosol-forming member 10a has been activated and aerosol has formed in the channels 25, the aerosol is drawn through the channels 25 as the user continues to inhale. The aerosol then exits the aerosol chamber 6 through a chamber outlet 31 provided in the housing 2 as seen in figure 1b. The aerosol then passes through an optional aerosol refining member 32 provided in the housing 2, causing the aerosol to be cooled. The refining member 32 may also contain flavouring agents like menthol that are released into the flow of aerosol before entering the user's mouth via the outlet 7 provided in the mouthpiece 3. Meanwhile, the solution that has evaporated from the capillary structure of the sheet of material is replaced by fresh solution from the reservoir 9 due to the capillary effect of the capillary gaps 17, 18 and the capillary structure of the aerosol-forming member 10a as described above and fresh air enters the channels 25 via the air inlet 5 and passage 4. In one embodiment, a pressure drop element/ flow resistor 33 is positioned in the passage 4 so that the flow of air into the aerosol chamber 6 can be controlled. The flow resistor 33 may consist of a simple aperture or hole and may be identical with the air inlet 5 in the housing 2. Alternatively, the flow resistor 33 may consist of a porous body similar to a cigarette filter providing the flow resistance of a conventional cigarette.

The valve 33 may be controlled by the PCB or manually, for example, by adjusting a switch or dial (not shown) on the housing 2 of the aerosol delivery device 1.

The degree of corrugation can be varied by varying the number of corrugations per distance unit. For example, in one embodiment the sheet of material comprises three corrugations per distance unit. In another embodiment the sheet of material comprises 6 corrugations per distance unit. The greater the degree of corrugation the more aerosol is generated by the aerosol-forming member per inhalation.

It should be understood that due to the corrugations of the above described embodiments, the aerosol-forming member has larger surface area compared to a flat aerosol-forming member. Advantageously, this increases the efficiency of the aerosol forming member 10a in that it can produce more aerosol per inhalation. Furthermore, due to the corrugation of the aerosol-forming member 10a, the aerosol delivery device 1 can be made more compact.

Referring now to figure 3a, another embodiment of an aerosol delivery device 51 is disclosed. Figure 3a shows a cross-section of an aerosol delivery device 51 similar to the aerosol delivery device 1 shown in figure 1a. The aerosol delivery device 51 comprises a housing 52 with a mouthpiece (not shown). A passage (not shown) is provided in the housing 52 and is open to atmosphere via an air inlet (not shown). The passage is in fluid communication with an aerosol chamber 56 which in turn is in fluid communication with an outlet aperture (not shown) formed in the mouthpiece. Therefore, in use, air may be drawn through the passage and into the aerosol chamber 56, via the passage, and then through the outlet aperture similar to the airflow of the aerosol delivery device shown in figure 1a.

An aerosol-forming member 60a is located in the aerosol chamber 56 as seen in figure 3a. The aerosol-forming member 60a comprises a sheet of material having corrugations 60b such that it forms peaks and troughs. A corrugation is to be understood as two bends, or a peak and a trough, or a ridge and a groove. The sheet of material may also be described as having a cross-sectional profile comprising at least one point of inflection.

The corrugations may follow a meandering or oscillating path, or a sine curve, or a zigzag-like configuration, or any other similar pattern. The aerosol-forming member 60a may comprise regular corrugations such that it comprises a repetition of identical corrugations as seen in figure 3a. However, in an alternative un-illustrated embodiment the aerosol-forming member may comprise irregular corrugations wherein the shape of peaks and troughs differ from one another. Although figure 3a shows an aerosol-forming member 60a comprising rounded corrugations 60b, i.e. rounded peaks and troughs, it should be understood that the invention is not limited thereto, but also includes corrugations forming vertices. These vertices may have obtuse, acute and/or right angles. This embodiment is shown in figure 3b and the reference numerals of figure 3a apply correspondingly to the components of figure 3b.

The aerosol-forming member 60a is similar to the embodiments of the aerosol-forming member 10a described above with reference to figures 1 and 2 and so a detailed description will be omitted. However, it should be understood that the aerosol-forming member 60a comprises a sheet material having two opposing major surfaces 66, 67. The aerosol-forming member 60a has an open-pored structure, foam structure or interconnecting network of pores, all of which form a capillary structure. The capillary structure enables the aerosol-forming member 60a to wick or absorb a solution. The sheet of material may comprise a single or a plurality of layers according to the various embodiments described with reference to figure 2.

The aerosol chamber 56 is defined by chamber walls comprising two opposing chamber side walls 53, 54 and two opposing chamber main walls 57a, 57b. The chamber main walls 57a, 57b comprise a liquid reservoir matrix 58, 59. The liquid reservoir matrix 58, 59 comprises a capillary structure, for example an interconnecting porous or open-porous structure, such that it can hold a solution or liquid. The liquid reservoir matrix 58, 59 comprises a heat resistant layer 62 and a resilient layer 63. The heat resistant layer 62 of each chamber main wall 57a, 57b is exposed to the space of the aerosol chamber 56 and the resilient layer of each main wall 57a, 57b is sandwiched between the heat resistant layer 62 and the housing 52.

The heat resistant layer 62 is sheet-like in nature and comprises one or a plurality of layers that may have a structure of fabric, mesh, woven fibre web, non-woven fibre web or foam. The heat resistant layer 62 is made of a heat resistant material, for example, glass, metal, carbon-based materials, ceramic, cotton or heat resistant plastic. If the heat resistant layer 62 is made of metal, the metal may be coated or oxidised so as to prevent short circuits. The heat resistant layer 62 is configured to resist heat so that it can withstand heat emitted by the aerosol forming member 60a and thereby protect the resilient layer 63.

The resilient layer 63 may comprise various structures, for example, its structure may be formed from woven fibres, non-woven fibres, foam or sponge. The resilient layer 63 may be made of plastic.

The resilient layer 63 provides a spring force such that it urges or biases the heat resistant layer 62 towards the corrugations 60b, i.e. the peaks, troughs or vertices, such that the heat resistant layer 62 of each liquid reservoir matrix 58, 59 is in contact with the corrugations, i.e. the peaks, troughs or vertices. This enables the liquid reservoir matrices 58, 59 to supply solution held therein to the aerosol-forming member 60a such that the solution spreads throughout the capillary structure of the aerosol-forming member 60a.

The capillarity of the aerosol-forming member 60a may be greater than the capillarity of the reservoir matrices 58, 59, but at least greater than the capillarity of the resilient layer 63 so as to induce a flow of solution from the liquid reservoir matrices 58, 59 towards the aerosol-forming member 60a. The capillarity is defined by the pore size and the wetting conditions of the respective capillary structures.

It should be understood that the present invention is not limited to comprising two liquid reservoir matrices 58, 59. It may comprise more than two liquid reservoir matrices. For example, each peak and trough of a corrugation may be in contact with discrete liquid reservoir matrices. In an alternative embodiment, only one chamber main wall 57a comprises a liquid reservoir matrix and the other chambermain wall 57b is made of a non-porous material (vice versa).

Ends 64, 65 of the aerosol-forming member 60a are attached to the chamber side walls 53, 54. Preferably both ends 64, 65 of the aerosol-forming member 60a are attached and preferably electrically connected to a support plate, which may be a printed circuit board (PCB). Alternatively, ends of the aerosol-forming member 60a may be attached to one of the heat resistant layers 62 of the chamber main walls 57a, 57b as seen in figure 3. In each embodiment, the aerosol-forming member 60a is suspended across the aerosol chamber 56.

The aerosol-delivery device 51 further comprises a battery (not shown) and a printed circuit board (PCB) (not shown) as described with reference to figures 1 and 2, and the aerosol-delivery device 51 is configured similar to the aerosol delivery device 1 described with reference to figures 1 and 2 such that the ends 64, 65 of the aerosol-forming member 60a, are electrically connected to the positive and negative terminals of the battery respectively. When current is drawn from the battery through the sheet of material of the aerosol-forming member 60a, the resistance of the sheet of material causes it to increase in temperature.

Operation of the aerosol-forming member 60a will now be described with reference to figure 3. Similar to the aerosol-delivery device 1 described with reference to figure 2, the user may manually activates the aerosol delivery device 51 or the aerosol delivery device 51 may be activated automatically as the user starts puffing on the aerosol delivery device 51. This may be achieved by a pressure sensor (not shown) located in the passage extending between the air inlet and aerosol chamber. In either embodiment, the battery provides a potential difference between the ends 64, 65 of the aerosol-forming member 60a as the aerosol delivery device 51 is activated, causing current to flow between the ends 64, 65 such that the sheet of material increases in temperature. This increase in temperature causes the solution held in the capillary structure of the sheet of material to evaporate so as to form a vapour. The vapour mixes with air drawn into the aerosol delivery device via passage by the user. The vapour mixes with air in the aerosol chamber 56, and as this occurs the vapour condenses and forms droplets such that an inhalable aerosol is produced.

The aerosol-forming member according to any of the above described embodiments with reference to figure 3 is located in the housing 52 such that the planes of the major surfaces 66, 67 are substantially parallel or aligned with the direction of the airflow. Thus, when a solution is held in the aerosol-forming member 60a and it is heated up such that the solution evaporates, the solution evaporates in a direction transverse to the direction of the airflow. In the embodiments wherein the capillary structure is exposed on both sides of the sheet of material, the solution is evaporated from both sides in opposite directions. The corrugations 60b of the sheet of material form channels 68 through which air flows. Furthermore, evaporated solution or vapour mixes with the airflow in the channels 68 such that aerosol is formed. Thus, the corrugations 60b or channels 68 direct the flow of aerosol through the aerosol delivery device 51 towards the user. Furthermore, as the sheet of material comprises corrugations solution is evaporated from the major surfaces 66, 67 in a direction towards another region of the major surfaces which results in reduced levels of aerosol condensing on the chamber walls and other internal components. Furthermore, as the aerosol-forming member 60a cools down aerosol and vapour remaining in the aerosol chamber 56 that condenses onto one of the major surfaces 66, 67 will be reabsorbed into the capillary structure of the aerosol-forming member 60a and re-evaporated as the aerosol-forming member 60a heats up again. Condensate formed on the chamber walls may at least partially be reabsorbed into the capillary structure of the heat resistant layer 62 and that way resupplied to the capillary structure of the aerosol-forming member 60a.

Similar to the aerosol-forming member 10a described with reference to figure 2, the corrugated configuration of the aerosol-forming member 60b shown in figure 3 also reduces condensation from forming on the chamber walls, internal components and/or inner walls of the housing 52. Thus, separate sponges other than the liquid reservoir matrices 58,59 or other extra means for absorbing condensation not inhaled by the user that are used in some conventional aerosol delivery devices may be omitted. This results in a more compact aerosol delivery device 51, as well as a simplified manufacturing process and reduced costs. Furthermore, by reducing the amount of aerosol from condensing onto the inner walls of the housing 52, the transfer of condensation heat to the housing 52 may be reduced, making the aerosol delivery device 51 more comfortable for the user to hold.

After the aerosol-forming member 60a has been activated and aerosol has formed in the channels 68, the aerosol is drawn through the channels 68 as the user continues to inhale. The aerosol then exits the aerosol chamber 56 through a chamber outlet provided in the housing 52. The aerosol then passes through an optional filter sponge provided in the housing 52, causing any large particulate in the flow to condense and be removed from the air flow before entering the user's mouth via the outlet provided in the mouthpiece. Meanwhile, the solution that has evaporated from the capillary structure of the sheet of material is replaced by fresh solution from the liquid reservoir matrices 58, 59 due to the capillary effect of the capillary structure and the peaks, troughs and/or vertices being in contact with the liquid reservoir matrices 58, 59. Fresh air enters the channel 68 via the air inlet and passage. In one embodiment, a flow resistor (as described above) is positioned in the passage so that the flow of air into the aerosol chamber 56 can be controlled. The valve may be controlled by the PCB or manually, for example, by adjusting a switch or dial (not shown) on the housing 52 of the aerosol delivery device 51.

It should be understood that due to the corrugation of the above described embodiments, the aerosol-forming member has larger surface area compared to a flat aerosol-forming member. Advantageously, this increases the efficiency of the aerosol forming member 10a in that it can produce more aerosol per inhalation. Furthermore, due to the corrugation of the aerosol-forming member 60a, the aerosol delivery device 51 can be made more compact.

Furthermore, the degree of corrugation of the aerosol-forming member 60a can be changed as described with reference to figure 2.

In any of the above mentioned embodiments of the aerosol-forming member, one or both of the ends 15, 16, 64, 65 may be located in line with or off-set from the peaks, troughs or vertices of the corrugations 10b, 60b.

In any of the above mentioned embodiments of the aerosol-forming component or device, there may be multiple aerosol-forming members, e.g. two, three, four, five or six aerosol-forming members. Where there are multiple aerosol-forming members, the number and configuration of the corrugations on each of the aerosol forming members may be the same or different. Where the number and configuration of the corrugations are the same, the aerosol-forming members may be positioned in the aerosol-forming component or device such that the corrugations are aligned. Where the multiple aerosol-forming members contain a different number and/or configuration of corrugations, the corrugations that are present may still be aligned. For example, two corrugations a first aerosol-forming member may be aligned with three corrugations of a second aerosol forming member. Such a configuration would result in the channels 25, 68 being maintained throughout the aerosol chamber. Alternatively, the multiple aerosol-forming members may be positioned in the aerosol-forming component or device such that the corrugations are offset. This may result in better mixing conditions between the vapour and the air and increased aerosol yields. The multiple aerosol forming members may be electrically connected in series or in parallel. Furthermore, the multiple aerosol forming members may be controlled differently, e.g. heated up sequentially.

The above described embodiments of the aerosol-forming member of the aerosol delivery device 1 are described for use with a solution. It should be understood that this solution may comprise certain constituents or substances that may have a stimulatory effect on the user. These constituents or substances may be of any kind that is suitable for being delivered via inhalation. The solution in which the constituents or substances are held or dissolved may primarily consist of water, ethanol, glycerol, propylene glycol or mixtures of the aforementioned solvents. By means of a sufficiently high degree of dilution in an easily volatile solvent, such as ethanol and/or water, even substances which are otherwise difficult to evaporate can evaporate in a substantially residue-free manner, and thermal decomposition of the liquid material can be avoided or significantly reduced.

In order to address various issues and advance the art, the entirety of this disclosure shows by way of illustration various embodiments in which the claimed invention(s) may be practiced and provide for superior aerosol-forming member, aerosol delivery device component and aerosol delivery device. The advantages and features of the disclosure are of a representative sample of embodiments only, and are not exhaustive and/or exclusive. They are presented only to assist in understanding and teach the claimed features. It is to be understood that advantages, embodiments, examples, functions, features, structures, and/or other aspects of the disclosure are not to be considered limitations on the disclosure as defined by the claims or limitations on equivalents to the claims, and that other embodiments may be utilised and modifications may be made without departing from the scope of the disclosure. Various embodiments may suitably comprise, consist of, or consist essentially of, various combinations of the disclosed elements, components, features, parts, steps, means, etc. In addition, the disclosure includes other inventions not presently claimed, but which may be claimed in future.

## Claims

1. An aerosol-forming member (10a, 60a) for an aerosol delivery device (1) comprising a sheet of material configured to heat and wick a solution, wherein the sheet of material comprises at least one corrugation (10b, 60b), so as to provide the sheet with a cross-sectional profile comprising at least one point of inflection.

2. An aerosol-forming member according to claim 1, wherein the sheet of material comprises a capillary structure configured to wick a solution.

3. An aerosol-forming member according to any one of the preceding claims, wherein the capillary structure extends throughout the whole sheet of material.

4. An aerosol-forming member according to claim 1, wherein the sheet of material comprises a first layer that is heatable and a second layer comprising a capillary structure.

5. An aerosol-forming member according to any one of the preceding claims, wherein the sheet of material comprises a peak and a trough.

6. An aerosol-forming member according to any one of the preceding claims, wherein the corrugation (10b, 60b) forms vertices or wherein the corrugation (10b, 60b) is rounded.

7. An aerosol delivery device component (1') comprising an air inlet (5) and an air outlet (7) fluidly communicating via an aerosol chamber (6, 56) defined by chamber walls, and an aerosol-forming member (10a, 60a) according to any of claims 1 to 6, the aerosol forming member (10a, 60a) being located at least partially within the aerosol chamber (6, 56).

8. An aerosol delivery device component according to claim 7, wherein the sheet of material comprises two opposing major surfaces (13, 14, 66, 67) that are aligned with a direction of flow of air through the aerosol chamber (6, 56).

9. An aerosol delivery device component according to claim 7 or 8, wherein the sheet of material comprises two opposing ends (15, 16, 64, 65) that are attached to the aerosol delivery device component such that the sheet of material is suspended across the aerosol chamber.

10. An aerosol delivery device component according to any of claims 7 to 9, wherein the at least one corrugation (10b, 60b) of the sheet of material is in close proximity to or is in contact with at least one of the chamber walls.

11. An aerosol delivery device component according to claim 7, wherein at least one of the chamber walls comprises a liquid reservoir matrix (9, 58, 59).

12. An aerosol delivery device component according to claim 11, wherein the liquid reservoir matrix (9, 58, 59) comprises a capillary structure.

13. An aerosol delivery device component according to claim 11 or 12, wherein the at least one corrugation (10b, 60b) of the sheet of material is in contact with the liquid reservoir matrix (9, 58, 59).

14. An aerosol delivery device (1, 51) comprising an aerosol delivery device component as claimed in claims 7 to 13 or aerosol-forming member as claimed in any of claims 1 to 6.

15. An aerosol delivery device according to claim 14 comprising multiple aerosol forming members, wherein the multiple aerosol forming members are positioned in the aerosol-forming component or device such that the corrugations (10b, 60b) are aligned with respect to the direction of airflow through the device or such that the corrugations are off-set with respect to the direction of airflow through the device.

## Patentansprüche

1. Aerosolbildendes Element (10a, 60a) für eine Aerosolabgabevorrichtung (1), umfassend ein Materialflächenstück, das zum Erwärmen und Aufsaugen einer Lösung ausgelegt ist, wobei das Materialflächenstück mindestens eine Riffelung (10b, 60b) umfasst, um dem Flächenstück ein Querschnittsprofil zu verleihen, das mindestens einen Wendepunkt umfasst.

2. Aerosolbildendes Element nach Anspruch 1, wobei das Materialflächenstück eine Kapillarstruktur umfasst, die zum Aufsaugen einer Lösung ausgelegt ist.

3. Aerosolbildendes Element nach einem der vorhergehenden Ansprüche, wobei sich die Kapillarstruktur durch das gesamte Materialflächenstück erstreckt.

4. Aerosolbildendes Element nach Anspruch 1, wobei das Materialflächenstück eine erste Schicht, die erwärmbar ist, und eine zweite Schicht, die eine Kapillarstruktur hat, umfasst.

5. Aerosolbildendes Element nach einem der vorhergehenden Ansprüche, wobei das Materialflächenstück eine Spitze und einen Tiefpunkt umfasst.

6. Aerosolbildendes Element nach einem der vorhergehenden Ansprüche, wobei die Riffelung (10b, 60b) Scheitelpunkte bildet oder wobei die Riffelung (10b, 60b) gerundet ist.

7. Bauteil (1') einer Aerosolabgabevorrichtung, umfassend einen Lufteinlass (5) und einen Luftauslass (7), die über eine von Kammerwänden definierte Aerosolkammer (6, 56) in Fluidverbindung stehen, und ein aerosolbildendes Element (10a, 60a) nach einem der Ansprüche 1 bis 6, wobei das aerosolbildende Element (10a, 60a) zumindest teilweise innerhalb der Aerosolkammer (6, 56) angeordnet ist.

8. Bauteil einer Aerosolabgabevorrichtung nach Anspruch 7, wobei das Materialflächenstück zwei gegenüberliegende Hauptflächen (13, 14, 66, 67) umfasst, die mit einer Richtung des Luftflusses durch die Aerosolkammer (6, 56) ausgerichtet sind.

9. Bauteil einer Aerosolabgabevorrichtung nach Anspruch 7 oder 8, wobei das Materialflächenstück zwei gegenüberliegende Enden (15, 16, 65, 65) umfasst, die so an dem Bauteil einer Aerosolabgabevorrichtung befestigt sind, dass das Materialflächenstück über der Aerosolkammer aufgehängt ist.

10. Bauteil einer Aerosolabgabevorrichtung nach einem der Ansprüche 7 bis 9, wobei die mindestens eine Riffelung (10b, 60b) des Materialflächenstücks in unmittelbarer Nähe mindestens einer der Kammerwände angeordnet oder damit in Kontakt ist.

11. Bauteil einer Aerosolabgabevorrichtung nach Anspruch 7, wobei mindestens eine der Kammerwände eine Flüssigkeitsreservoirmatrix (9, 58, 59) umfasst.

12. Bauteil einer Aerosolabgabevorrichtung nach Anspruch 11, wobei die Flüssigkeitsreservoirmatrix (9, 58, 59) eine Kapillarstruktur umfasst.

13. Bauteil einer Aerosolabgabevorrichtung nach Anspruch 11 oder 12, wobei die mindestens eine Riffelung (10b, 60b) des Flächenmaterialstücks mit der Flüssigkeitsreservoirmatrix (9, 58, 59) in Kontakt ist.

14. Aerosolabgabevorrichtung (1, 51), umfassend ein Bauteil einer Aerosolabgabevorrichtung nach den Ansprüchen 7 bis 13 oder ein aerosolbildendes Element nach einem der Ansprüche 1 bis 6.

15. Aerosolabgabevorrichtung nach Anspruch 14, umfassend mehrere aerosolbildende Elemente, wobei die mehreren aerosolbildenden Elemente in dem aerosolbildenden Bauteil oder der Vorrichtung angeordnet sind, so dass die Riffelungen (10b, 60b) bezüglich der Richtung der Luftströmung durch die Vorrichtung ausgerichtet sind oder so dass die Riffelungen bezüglich der Richtung der Luftströmung durch die Vorrichtung versetzt sind.

## Revendications

1. Élément de formation d'aérosol (10a, 60a) pour un dispositif de distribution (1) d'aérosol comprenant une feuille de matériau conçue pour chauffer et absorber par effet de mèche une solution, la feuille de matériau comprenant au moins une ondulation (10b, 60b), de façon à doter la feuille d'un profil en coupe transversale comprenant au moins un point d'inflexion.

2. Élément de formation d'aérosol selon la revendication 1, dans lequel la feuille de matériau comprend une structure capillaire conçue pour absorber par effet de mèche une solution.

3. Élément de formation d'aérosol selon l'une quelconque des revendications précédentes, dans lequel la structure capillaire s'étend sur toute la feuille de matériau.

4. Élément de formation d'aérosol selon la revendication 1, dans lequel la feuille de matériau comprend une première couche qui peut être chauffée et une seconde couche comprenant une structure capillaire.

5. Élément de formation d'aérosol selon l'une quelconque des revendications précédentes, dans lequel la feuille de matériau comprend un pic et un creux.

6. Élément de formation d'aérosol selon l'une quelconque des revendications précédentes, dans lequel l'ondulation (10b, 60b) forme des sommets ou dans lequel l'ondulation (10b, 60b) est arrondie.

7. Composant (1') de dispositif de distribution d'aérosol comprenant une admission (5) d'air et une évacuation (7) d'air en communication fluidique par le biais d'une chambre (6, 56) d'aérosol délimitée par des parois de chambre, et un élément (10a, 60a) de formation d'aérosol selon l'une quelconque des revendications 1 à 6, l'élément (10a, 60a) de formation d'aérosol étant situé au moins partiellement dans la chambre (6, 56) d'aérosol.

8. Composant de dispositif de distribution d'aérosol selon la revendication 7, dans lequel la feuille de matériau comprend deux surfaces principales opposées (13, 14, 66, 67) qui sont alignées sur un sens de flux d'air à travers la chambre (6, 56) d'aérosol.

9. Composant de dispositif de distribution d'aérosol selon la revendication 7 ou 8, dans lequel la feuille de matériau comprend deux extrémités opposées (15, 16, 64, 65) qui sont fixées au composant de dispositif de distribution d'aérosol de sorte que la feuille de matériau soit suspendue en travers de la chambre d'aérosol.

10. Composant de dispositif de distribution d'aérosol selon l'une quelconque des revendications 7 à 9, dans lequel l'au moins une ondulation (10b, 60b) de la feuille de matériau est au voisinage immédiat d'au moins une des parois de chambre ou en contact avec au moins une de celles-ci.

11. Composant de dispositif de distribution d'aérosol selon la revendication 7, dans lequel au moins une des parois de chambre comprend une matrice (9, 58, 59) de réservoir de liquide.

12. Composant de dispositif de distribution d'aérosol selon la revendication 11, dans lequel la matrice (9, 58, 59) de réservoir de liquide comprend une structure capillaire.

13. Composant de dispositif de distribution d'aérosol selon la revendication 11 ou 12, dans lequel l'au moins une ondulation (10b, 60b) de la feuille de matériau est en contact avec la matrice (9, 58, 59) de réservoir de liquide.

14. Dispositif de distribution (1, 51) d'aérosol comprenant un composant de dispositif de distribution d'aérosol selon les revendications 7 à 13 ou un élément de formation d'aérosol selon l'une quelconque des revendications 1 à 6.

15. Dispositif de distribution d'aérosol selon la revendication 14 comprenant de multiples éléments de formation d'aérosol, les multiples éléments de formation d'aérosol étant positionnés dans le composant de formation d'aérosol ou le dispositif de sorte que les ondulations (10b, 60b) soient alignées par rapport au sens du flux d'air à travers le dispositif ou de sorte que les ondulations soient décalées par rapport au sens du flux d'air à travers le dispositif.
